# EUROPÄISCHE PATENTANMELDUNG

(11) **EP 0 979 663 A2**
(43) Veröffentlichungstag der Anmeldung: **16.02.2000**
(21) Anmeldenummer: 99108840.2
(22) Anmeldetag: 04.05.1999
(51) Int. Cl.: A61M 16/20

(54) **Narkosevorrichtung**

(30) Priorität: 11.08.1998 DE 29814375 U
(71) Anmelder: Füllekrug, Bernd Dr., 20251 Hamburg (DE); Beyer, Rudolf, 22301 Hamburg (DE)
(72) Erfinder: Füllekrug, Bernd Dr., 20251 Hamburg (DE); Beyer, Rudolf, 22301 Hamburg (DE)
(74) Vertreter: Selting, Günther, Dipl.-Ing.

(57) **Zusammenfassung**

Die Narkosevorrichtung weist einen Inspirationsschlauch (10) mit Inspirationsventil (19) und einen Exspirationsschlauch (11) mit Exspirationsventil (14) auf. Diese Schläuche sind patientenseitig mit einem Verbindungsstück (12) verbunden, welches üblicherweise als Y-Stück ausgebildet ist. Erfindungsgemäß ist das Verbindungsstück (12) als Wegeventil gestaltet und mit einem Auslaßanschluß (26) versehen. Das Wegeventil ist so ausgebildet, daß entweder der Auslaßanschluß (26) oder der Exspirationsanschluß (24) verschlossen wird. Auf diese Weise besteht die Möglichkeit, eine Beatmung wahlweise im halbgeschlossenen Kreissystem oder im halbgeöffneten Kreissystem durchzuführen.

## Beschreibung

Die Erfindung betrifft eine Narkosevorrichtung zur Narkotisierung und Beatmung von Patienten, und insbesondere eine Narkosevorrichtung, bei der ein Inspirationsschlauch und ein Exspirationsschlauch durch ein Verbindungsstück miteinander und mit einem Patientenanschluß verbunden sind.

Die Beatmung von Patienten kann unter anderem mit einem halboffenen Kreissystem, mit einem halbgeschlossenen oder einem geschlossenen Kreissystem erfolgen. Bei einem halboffenen Kreissystem atmet der Patient sauerstoffhaltiges Gas aus einer Atemgasquelle ein, wobei die Ausatmung in die Umgebungsluft erfolgt. Diese Art der Beatmung erfordert eine große Menge an Atemgas, bietet aber den Vorteil einer sehr guten Frischgasversorgung des Patienten. Bei einem halbgeschlossenen System erfolgt die Ausatmung in ein Kreissystem. Dieses ist an einen CO₂-Absorber angeschlossen, der außerdem mit einer Frischgasleitung verbunden ist. In dem Absorber wird das Gemisch aus vom Patienten ausgeatmeter Luft und frischer Atemluft gemischt und gereinigt und dem Patienten zugeführt. Durch den in dem Ausatemgas enthaltenen Restsauerstoff wird der Verbrauch an frischem Atemgas verringert. Weit überwiegend wird in Narkosevorrichtungen mit halbgeschlossenem Kreislauf gearbeitet.

Die Injektion hochpotenter Narkotika, wie sie zur Einleitung einer Vollnarkose gegeben werden, induziert einen Atemstillstand, der die Gefahr einer hypoxischen Schädigung des Patienten bedeuten kann. Der Hintergrund dafür ist, daß ein erheblicher Teil der anästhesiologischen Mortalität auf eine Hypoxie zurückgeführt werden kann, die nach der Induktion eines Atemstillstandes nicht beherrscht wird ("can not intubate - can not ventilate"-Situation). Dies trifft vor allem auf pulmonale und cardiale Risikopatienten zu, deren Kompensationsmöglichkeiten gegenüber Störungen im Sauerstoffhaushalt bereits ohne Vollnarkose nahezu erschöpft sind. Darüber hinaus sind häufig Säuglinge und Kleinkinder, Schwangere und vor allem adipöse Patienten durch eine auch nur kurzfristige Unterbrechung der Sauerstoffzufuhr gefährdet. Da bei jeder Vollnarkose ein künstlicher Atemstillstand erzeugt wird und generell keine definitive Vorhersage über die Zeit bis zur Sicherung der Atemwege mit Maske, Tubus oder Kehlkopfmaske möglich ist, bedeutet eine maximale Sauerstoffspeicherung in der Lunge einen erheblichen Sicherheitsgewinn, weil der hierbei angelegte intrapulmonale Sauerstoffspeicher eine ausreichende Sauerstoffversorgung der Zellen für mehrere Minuten bedeutet. Die Präoxygenierung ist Standard bei der Narkoseeinleitung und wird üblicherweise mit einem mit Sauerstoff gefüllten Kreislauf (Kreisteil) und auf das Gesicht des Patienten aufgesetzter Gesichtsmaske durchgeführt, wobei die Maske über ein Y-Stück mit dem Ein- und dem Ausatemschenkel des Kreislaufs verbunden ist. Selbst über eine dichtsitzende Maske mit hohem Frischgasfluß ist jedoch eine optimale Präoxygenierung nur eingeschränkt möglich. Limitierende Faktoren sind u.a. die erreichbare inspiratorische Sauerstoffkonzentration, die durch die Rückatmung in einem halbgeschlossenen Kreislauf nur langsam 100 % erreicht.

Der Erfindung liegt die Aufgabe zugrunde, eine Narkosevorrichtung zu schaffen, die im halbgeschlossenen System betrieben werden kann und dabei die Möglichkeit einer Verbesserung der Präoxygenierung bietet und somit ein einfaches und effektives Mittel bildet, um die durch die Narkoseinduktion bedingte Morbidität und Mortalität zu senken.

Die Lösung dieser Aufgabe erfolgt erfindungsgemäß mit den im Patentanspruch 1 angegebenen Merkmalen.

Erfindungsgemäß enthält das Verbindungsstück, das die patientenseitigen Enden von Inspirationsschlauch und Exspirationsschlauch sowohl untereinander als auch mit dem Patientenanschluß verbindet, ein verstellbares Wegeventil, das zusätzlich einen in die Umgebung führenden Auslaßanschluß aufweist und so ausgebildet ist, daß es entweder den Exspirationsanschluß oder den Auslaßanschluß verschließt. Dabei ist es möglich, den noch wachen Patienten vor der Narkotisierung mit reinem Sauerstoff aus dem Kreissystem zu versorgen, indem das Kreissystem im halboffenen Zustand betrieben wird, wobei der Patient stets reines, nicht durch Ausatemgase verunreinigtes Atemgas erhält und in die Umgebung hinein ausatmet. Der noch wache, spontan atmende Patient kann somit reinen Sauerstoff aus dem Kreissystem einatmen und seine Stickstoff enthaltende Ausatemluft in die Umgebung abatmen. Wenn der Patient die Spontanatmung einstellt, kann der Narkosearzt das Wegeventil umschalten und den Patienten über das übliche halbgeschlossene Kreissystem manuell oder maschinell beatmen. Hierbei wird der Stickstoff aus der Lunge des Patienten in das halbgeschlossene Kreisteil ausgeatmet, wodurch der inspiratorische Sauerstoffanteil erniedrigt wird. Andererseits wird der Verbrauch an frischer Atemluft erheblich reduziert.

Mit einem üblichen halbgeschlossenen Kreissystem benötigt man bei einem Frischgasfluß von 10 l/min. je nach Patient im Mittel etwa 3 bis 5 min. bis zur maximalen Sauerstoffeinwaschung. Mit der beschriebenen Umschaltvorrichtung, die einen Betrieb im halboffenen Kreissystem patientennah ermöglicht, wird stets reiner Sauerstoff dem Patienten angeboten und so die Zeit bis zur vollständigen Präoxygenierung verkürzt. Dies erhöht die Sicherheit des Patienten bei der Narkoseeinleitung.

Gemäß einer bevorzugten Weiterbildung der Erfindung ist vorgesehen, daß der Auslaßanschluß mit einem nur in die Umgebung durchlässigen Rückschlagventil verbunden ist. Dieses Rückschlagventil bildet ein Nichtrückatem-Ventil, welches verhindert, daß der Patient Umgebungsluft einatmet. Die Verbindung der Narkosevorrichtung zum Patienten erfolgt durch eine mit dem Patientenanschluß verbundene Gesichtsmaske oder über einen Beatmungsschlauch (Tubus). Die Ventilvorrichtung kann auch fester Bestandteil von sogenannten Einmalschläuchen oder mehrfach verwendbaren Schläuchen für Narkosekreisteile sein. Auch eine Integration der Ventilvorrichtung in einen patientennahen Atemgasfilter bzw. Klimatisierungsfilter (HME-Filter) ist möglich und führt zu dem beschriebenen Effekt einer schnelleren Präoxygenierung.

Das Wegeventil ist vorzugsweise mit einem zylindrischen Gehäuse versehen, in dem ein ebenfalls zylindrisches Küken angeordnet ist. Von dem Gehäuse stehen die Anschlüsse für Inspiration und Exspiration sowie der Auslaßanschluß strahlenförmig ab, während der Patientenanschluß axial abgeht. Es ist aber auch eine andere Umschaltvorrichtung möglich, zum Beispiel eine Umschaltvorrichtung, die einen Linearschieber enthält.

Im folgenden wird unter Bezugnahme auf die Zeichnungen ein Ausführungsbeispiel der Erfindung näher erläutert.

Es zeigen:
- Fig. 1: eine schematische Darstellung der Narkosevorrichtung, wobei das Wegeventil auf ein halbgeschlossenes Kreissystem eingestellt ist,
- Fig. 2: die Narkosevorrichtung, wobei das Wegeventil auf ein halboffenes Kreissystem eingestellt ist,
- Fig. 3: eine perspektivische Ansicht des Verbindungsstückes bei halboffenem System während des Einatmens,
- Fig. 4: die gleiche Ventilstellung wie Fig. 3, jedoch während des Ausatmens,
- Fig. 5: das Verbindungsstück in der Stellung, die dem halbgeschlossenen System entspricht, während des Einatmens,
- Fig. 6: die gleiche Ventilstellung wie in Fig. 5, jedoch während des Ausatmens,
- Fig. 7: einen Längsschnitt durch das Wegeventil und
- Fig. 8: einen Schnitt entlang der Linie VIII-VIII von Fig. 7.

Die Narkosevorrichtung weist einen Inspirationsschlauch 10 zum Einatmen und einen Exspirationsschlauch 11 zum Ausatmen auf.

Beide Schläuche sind an ihrem patientenseitigen Ende durch ein Verbindungsstück 12 miteinander verbunden. An das Verbindungsstück 12 ist der Patient angeschlossen, hier über einen Beatmungsschlauch 13.

Das patientenferne Ende des Exspirationsschlauches 11 ist mit einem Exspirationsventil 14 verbunden, welches als Rückschlagventil ausgebildet ist und nur nach außen (aus dem Schlauch heraus) durchlässig ist, in Gegenrichtung jedoch sperrt. Das Exspirationsventil 14 ist mit einem Verbindungskanal 15 verbunden, an den ein Atembeutel 16 angeschlossen ist und in den eine Atemgasleitung 17 mündet, die von einer (nicht dargestellten) Atemgasquelle kommt. Der Verbindungskanal 15 ist ferner mit einem Absorber 18 verbunden, der das in dem Exspirationsgasgemisch enthaltene CO₂ absorbiert. Am Auslaß des Absorbers 18 bzw. am Einlaß des Inspirationsschlauches 10 ist ein Inspirationsventil 19 vorgesehen, das als Rückschlagventil ausgebildet ist, welches nur in Richtung zum Inspirationsschlauch 10 hin durchlässig ist.

An den Verbindungskanal 15 ist schließlich noch ein Überdruckventil 20 angeschlossen, welches bei Überschreiten eines Grenzdrucks im Verbindungskanal 15 in einen Narkosemittelfilter 21 oder Absauger hinein öffnet. Das Überdruckventil 20 kann durch Betätigen eines Handgriffs 22 abgesperrt werden.

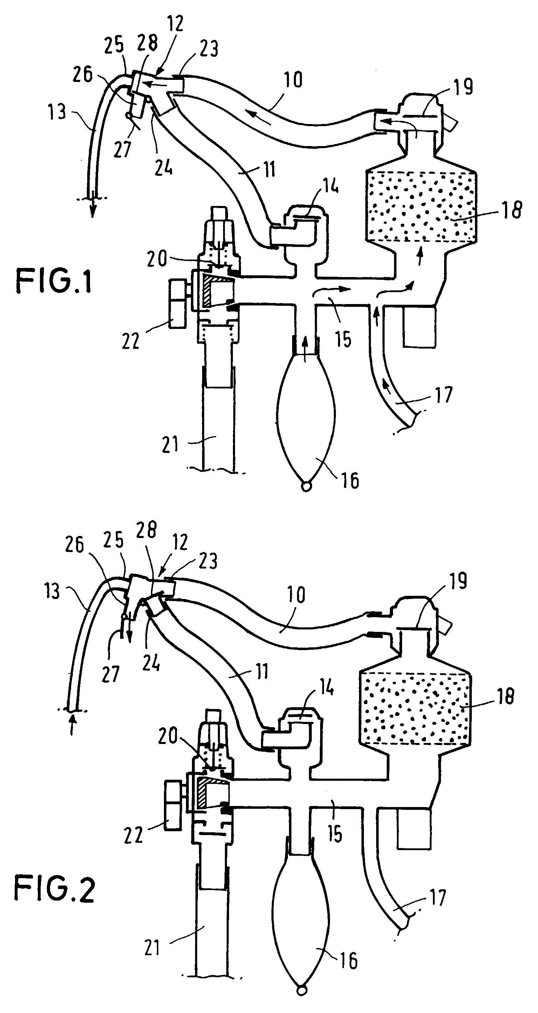

Das Verbindungsstück 12 weist einen Inspirationsanschluß 23, einen Exspirationsanschluß 24 und einen Patientenanschluß 25 auf. Zusätzlich ist erfindungsgemäß ein Auslaßanschluß 26 vorgesehen, der das Innere des Verbindungsstücks mit der Umgebung verbindet. Am Auslaßanschluß 26 befindet sich ein nur in die Umgebung öffnendes Rückschlagventil 27. Ferner enthält der Auslaßanschluß 26 ein Ventilorgan 28, welches in der in Fig. 1 dargestellten Position den Auslaßanschluß 26 verschließt und in der in Fig. 2 dargestellten Position den Exspirationsanschluß 24 verschließt.

Wenn die Narkosevorrichtung in dem in Fig. 1 dargestellten Zustand ist, der das halbgeschlossene Kreissystem kennzeichnet und bei dem der Auslaßanschluß 26 geschlossen ist, wird Ausatemluft des Patienten durch den Exspirationsschlauch 11 und das Exspirationsventil 14 hindurch in den Atembeutel 16 hinein ausgeatmet. Beim anschließenden Einatmen des Patienten vermischt sich das Ausatemluftgemisch im Atembeutel 16 mit dem frischen Atemgas aus der Atemgasleitung 17. Dieses Gemisch wird über den Absorber 18 und das Inspirationsventil 19 sowie durch den Inspirationsschlauch 10 vom Patienten eingeatmet.

Zur Einwaschung von Sauerstoff in die Patientenlunge wird das Ventilorgan 28 in den in Fig. 2 dargestellten Zustand eingestellt, wodurch die Narkosevorrichtung patientennah im halboffenen Kreissystem betrieben wird. Hierbei wird der Exspirationsanschluß 24 verschlossen, während der Auslaßanschluß 26 geöffnet wird. Somit wird die Ausatemluft des Patienten durch das in dieser Richtung offene Rückschlagventil 27 in die Umgebung abgelassen.

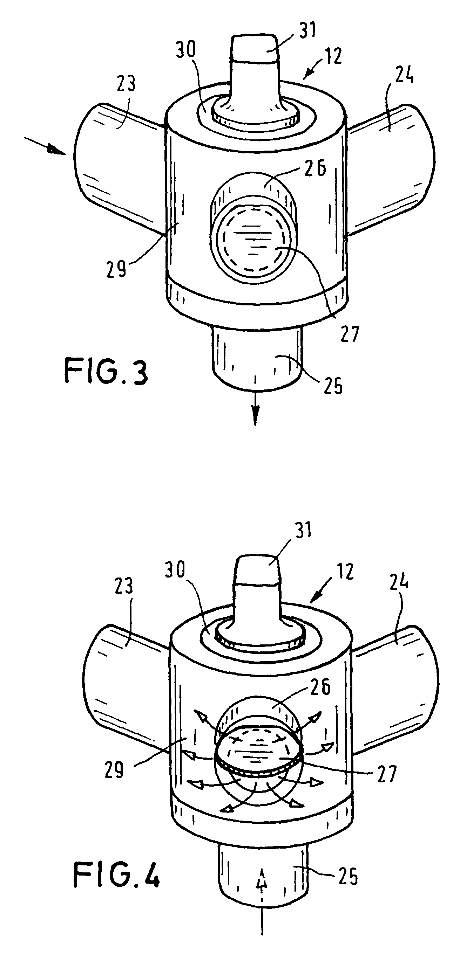

In den Fign. 3-6 sind perspektivische Ansichten des Verbindungsstücks 12 dargestellt. Dieses Verbindungsstück ist als Wegeventil ausgebildet. Es weist ein zylindrisches Gehäuse 29 auf, in dem ein Ventilküken 30 drehbar angeordnet ist. Das Ventilküken 30 ist mit einem Ventilgriff 31 versehen, der mit der Hand gedreht werden kann, um die Ventilstellung zu verändern.

Der Inspirationsanschluß 23, der Exspirationsanschluß 24 und der Auslaßanschluß 26 sind sternförmig verteilt um den Umfang des Gehäuses 29 angeordnet. Der Patientenanschluß 25 geht dagegen axial von einer Stirnseite des Gehäuses 29 ab. Der Patientenanschluß 25 ist ständig, also bei jeder Drehstellung des Ventilkükens 30, mit dem Inspirationsanschluß 23 verbunden.

In den Fign. 3 und 4 ist das Ventil in der Stellung, die dem halboffenen Kreissystem entspricht, dargestellt. Hierbei ist der Ventilinnenraum, der mit dem Inspiratiosanschluß 23 und dem Patientenanschluß 25 verbunden ist, auch mit dem Auslaßanschluß 26 verbunden. Fig. 3 zeigt den Zustand während der Inspiration. Hierbei saugt der Patient Atemgas durch den Inspirationsanschluß 23 und den Patientenanschluß 25 an. Das Ventilküken 30, welches dem Ventilorgan 28 der Fign. 1 und 2 entspricht, sperrt den Exspirationsanschluß 24 ab.

Beim Ausatmen gemäß Fig. 4 strömt die Ausatemluft vom Patientenanschluß 25 zum Auslaßanschluß 25, wo sie durch das Rückschlagventil 27 hindurch ins Freie strömt.

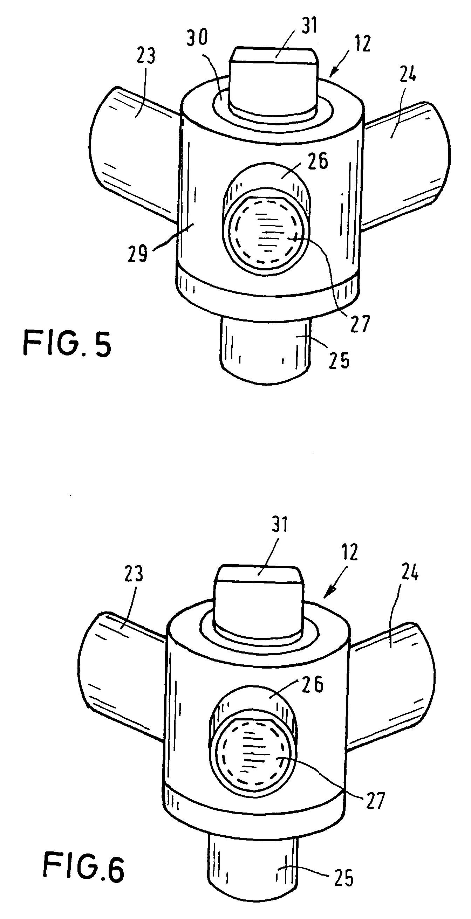

In den Fign. 5 und 6 ist das Verbindungsstück 12 in dem Zustand dargestellt, der dem halbgeschlossenen Kreissystem entspricht. Der Ventilgriff 31 ist gegenüber den Fign. 3 und 4 um 90° verdreht, so daß das Ventilküken 30 den Auslaßanschluß 26 bleibend versperrt, während es den Exspirationsanschluß 24 öffnet.

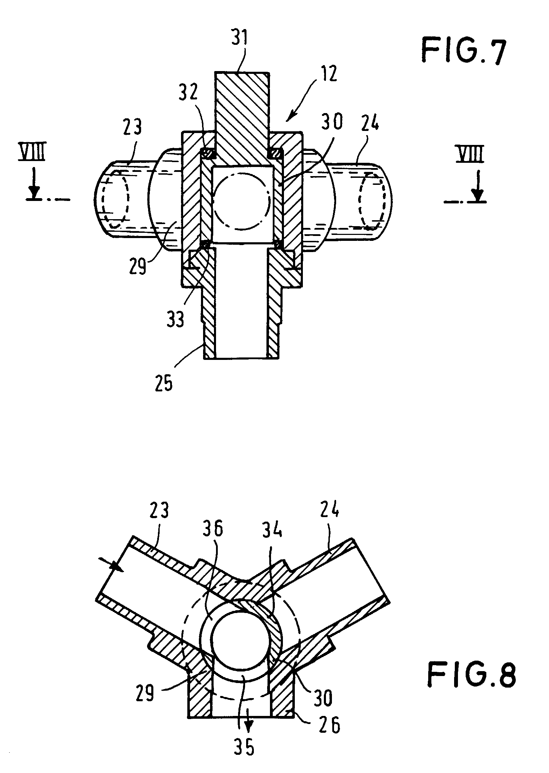

Aus den Fign. 7 und 8 ist der Aufbau des Verbindungsstücks im Detail ersichtlich. Das Ventilküken 30 ist in dem Gehäuse 29 drehbar gelagert und durch Dichtringe 32,33 an den Stirnseiten abgedichtet. Das Ventilküken 30 weist einen geschlossenen Umfangswandteil 34 und zwei kreissektorförmige Durchlässe 35,36 auf. In Fig. 8 ist der Zustand dargestellt, daß das Wandteil 34 den Exspirationsanschluß 24 verschließt, während die beiden Durchlässe 35,36 die anderen Anschlüsse 26,23 öffnen. Durch Drehen des Ventilkükens 30 kann alternativ der Auslaßanschluß 26 verschlossen werden, während die beiden anderen Anschlüsse 23,24 geöffnet werden. Das Ventilküken kann also zwei um 120° versetzte Drehpositionen einnehmen. Es hält den Inspirationsanschluß 23 ständig frei und verschließt abwechselnd den Exspirationsanschluß 24 oder den Auslaßanschluß 26.

## Patentansprüche

1. Narkosevorrichtung mit einem Inspirationsschlauch (10), der mit einem Inspirationsventil (19) verbunden ist, einem Exspirationsschlauch (11), der mit einem Exspirationsventil (14) verbunden ist, und einem den Inspirationsschlauch (10) und den Exspirationsschlauch (11) mit einem Patientenanschluß (25) verbindenden Verbindungsstück (12),
**dadurch gekennzeichnet**,
daß das Verbindungsstück (12) als umschaltbares Wegeventil ausgebildet ist, das einen Inspirationsanschluß (23), einen Exspirationsanschluß (24), einen Patientenanschluß (25) und einen in die Umgebung führenden Auslaßanschluß (26) aufweist und so ausgebildet ist, daß es den Exspirationsanschluß (24) oder den Auslaßanschluß (26) verschließt.

2. Narkosevorrichtung nach Anspruch 1, dadurch gekennzeichnet, daß der Auslaßanschluß (26) mit einem nur in die Umgebung öffnenden Rückschlagventil (27) verbunden ist.

3. Narkosevorrichtung nach Anspruch 1 oder 2, dadurch gekennzeichnet, daß das Verbindungsstück (12) ein zylindrisches Gehäuse (29) aufweist, von dem der Inspirationsanschluß (23), der Exspirationsanschluß (24) und der Auslaßanschluß (26) strahlenförmig abstehen, während der Patientenanschluß (25) axial abgeht.

4. Narkosevorrichtung nach einem der Ansprüche 1-3, dadurch gekennzeichnet, daß das Verbindungsstück (12) ein drehbares Ventilküken (30) enthält.
